# EUROPEAN PATENT APPLICATION

(11) **EP 4 826 739 A1**
(43) Date of publication of application: **21.10.2026**
(21) Application number: 25305561.0
(22) Date of filing: 16.04.2025
(51) Int. Cl.: G16H 50/70, G16H 50/20, A61B 5/349, A61B 5/00, G16H 50/30

(54) **ECG SIGNAL ANALYSIS**

(71) Applicant: Koninklijke Philips N.V., 5656 AE Eindhoven (NL)
(72) Inventor: KHELDOUNI, Amine, 5656 Eindhoven (NL); SHRESHTHA, Kumar, 5656 Eindhoven (NL); CHOFFIN, Benoît, 5656 Eindhoven (NL)
(74) Representative: Philips Intellectual Property & Standards

(57) **Abstract**

A method comprising accessing a clinical database recording historical ECG signal recordings and epidemiological data for a patient population, clustering waveform features extracted from the historical ECG signal recordings according to morphology characteristics to derive ECG waveform morphology clusters, correlating each morphology cluster with epidemiological information associated with corresponding patients, identifying epidemiological features correlated with each cluster, and creating a data structure associating ECG waveform feature morphology with correlated epidemiological features.

## Description

### FIELD OF THE INVENTION

The present invention relates to electrocardiogram (ECG) signal analysis and processing, and in particular to inference of clinically relevant information from ECG signal data.

### BACKGROUND OF THE INVENTION

Electrocardiograms (ECGs) are widely used diagnostic tools in cardiology, providing valuable information about the electrical activity of the heart. Traditional ECG interpretation relies on visual analysis by trained clinicians, which can be time-consuming and subject to variability. As the volume of ECG data continues to grow, there is an increasing need for automated analysis methods to assist clinicians in efficiently and accurately interpreting ECG signals.

There exist ECG analysis algorithms. However, current ECG analysis systems focus on detecting specific known cardiac conditions or abnormalities. Novel patterns and relationships between ECG morphology and patient health status that may not be readily apparent through conventional analysis methods.

### SUMMARY OF THE INVENTION

The invention is defined by the claims.

An aspect of the invention is a computer program product comprising computer program code configured, when run on a processor, to cause the processor to perform a method. The method comprises: accessing a clinical database recording historical ECG signal recordings for a patient population, and epidemiological data for the patient population; clustering waveform features extracted from the historical ECG signal recordings according to morphology characteristics to derive a plurality of ECG waveform morphology clusters; using the epidemiological data in the clinical database, correlating each of the plurality of morphology clusters with epidemiological information associated with the patients to which the ECG waveform features in each cluster correspond; identifying by the correlating one or epidemiological features correlated with each cluster; and creating a data structure associating ECG waveform feature morphology with correlated epidemiological features and transferring the data structure to a datastore.

Embodiments of the invention enable the creation of a data structure linking ECG waveform morphologies to epidemiological features, which can be used to identify potential health conditions or predispositions based on ECG patterns. This approach may for example leverage large-scale patient data to uncover correlations that may not be apparent through traditional ECG analysis methods.

**In** some embodiments, the method comprises a process of extracting one or more ECG waveform features from each of at least a subset of the historical ECG signal recordings, and performing the clustering of the extracted ECG waveform features. The process of extracting may comprise segmenting the ECG signals, wherein the ECG waveform features are ECG waveform segments,. **In** some embodiments, the process of extracting one or more waveform features may comprise ECG beat segmentation, wherein the ECG waveform features are individual ECG beats, or sections thereof. **In** some embodiments, the process of extracting one or more ECG waveform features may comprise identifying and segmenting individual ECG beats, for example based on using a delineation algorithm, and subsequently extracting one or more features from each individual beat, The extracted features may comprise morphological characteristics, temporal intervals, amplitude measurements, frequency-domain characteristics, or combinations thereof.

**In** some embodiments, the one or more epidemiological features include one or more of: a predictor of at least one extant health condition; a predictor of at least one future health condition; or a predictor of at least one predisposition to a health condition.

This feature enhances the clinical utility of the system by providing predictive capabilities for current and future health conditions, as well as identifying potential health risks based on ECG morphology.

**In** some embodiments, the method further comprises: receiving a measured ECG signal of a patient; matching each of one or more waveform features of the received ECG signal to one or more of the morphology clusters; and identifying, based on the matching, one or more epidemiological features correlated with the ECG signal of the patient.

This functionality allows the system to analyze new patient ECG data (optionally in real time) and provide insights based on the established correlations, potentially aiding in early detection or risk assessment of various health conditions.

**In** some embodiments, the method comprises applying ECG beat segmentation to each of the ECG signal recordings of the clinical database to extract individual ECG beats, and wherein the ECG waveform features correspond to individually segmented ECG beats, or sections thereof.

**In** some embodiments, the clustering comprises extracting from each waveform feature one or more predetermined morphological features and clustering the waveform features based on the extracted morphological features. The one or more pre-determined morphological features may comprise at least one of: P wave characteristics, QRS complex characteristics, and T wave characteristics. By focusing on specific, clinically relevant aspects of the ECG waveform, this approach can improve the accuracy and interpretability of the correlations between ECG morphology and epidemiological features.

**In** some embodiments, the method further comprises: responsive to an update of the clinical database, identifying one or more ECG signal recordings newly added to the database; extracting one or more waveform features from the one or more newly added ECG signal recordings and assigning the extracted waveform features to one or more of the morphology clusters based on morphology; and updating the correlated epidemiological information for the one or more morphology clusters. This feature allows the system to continuously learn and improve the correlations as new data becomes available, ensuring that the insights provided remain up-to-date and relevant.

In some embodiments, the method further comprises: responsive to an update of the clinical database, identifying one or more ECG signal recordings newly added to the database; extracting one or more waveform features from the one or more newly added ECG signal recordings; evaluating a morphology similarity measure between morphology of each of the extracted waveform features and an average morphology associated with each of the morphology clusters; and responsive to the similarity measure for a given extracted waveform feature being below a predetermined threshold for all of the morphology clusters, adjusting the morphology clusters to include a new morphology cluster which includes at least the given waveform feature. This adaptive clustering approach allows the system to identify and incorporate novel ECG morphologies that may be associated with previously unrecognized epidemiological features or health conditions not reflected in the existing set of morphology clusters.

In some embodiments, the method further comprises performing a cluster refinement operation comprising merging two or more morphology clusters or splitting one or more morphology clusters, optionally wherein the cluster refinement operation is performed responsive to a morphology similarity assessment applied to waveform features of the plurality of morphology clusters. This refinement process helps maintain the optimal balance between cluster granularity and meaningful correlations, improving the overall accuracy and usefulness of the system.

In some embodiments, the patient epidemiological data includes one or more of the following epidemiological features of each patient: age, sex, medical history, lifestyle factors, genetic information, and diagnosed health conditions. By incorporating a wide range of epidemiological data, the system can identify complex relationships between ECG morphology and various patient characteristics, potentially uncovering novel biomarkers or risk factors.

In some embodiments, the method further comprises generating a software module having a query processing function which uses the generated data structure, wherein the software module is operable to receive a query indicative of a waveform feature morphology and is operable to output a response indicative of one or more correlated epidemiological features, using the generated data structure. This query functionality allows healthcare providers to easily access and utilize the insights generated by the system, potentially improving clinical decision-making and patient care.

In some embodiments, the method comprises: receiving an ECG signal; displaying the received ECG signal using a user interface; receiving from the user interface indication of a user-indicated waveform feature comprised by the ECG signal; querying the software module based on the user-indicated waveform feature; receiving a response from the software module; and displaying using the user interface an indication of one or more epidemiological features correlated with the waveform feature based on the response. This interactive interface allows clinicians to explore and interpret the correlations between specific ECG features and epidemiological characteristics, potentially leading to more informed diagnoses and treatment decisions.

In some embodiments, the user interface is configured to detect the user-indicated waveform feature based on detecting a user cursor hover above the waveform feature of the displayed ECG signal. This intuitive interaction method simplifies the process of querying the system, making it more accessible and efficient for healthcare providers to use in clinical settings.

In some embodiments, the method further comprises: matching the user-indicated waveform feature to one of the morphology clusters; determining an average morphology waveform for the matching morphology cluster; and using the user interface, displaying the average morphology waveform as an overlay superimposed atop the user-indicated waveform feature. This visual comparison feature aids in the interpretation of individual ECG features by providing context within the broader morphology cluster, potentially highlighting subtle deviations or similarities that may be clinically relevant.

A further aspect of the invention is a processing system comprising one or more processors configured to execute the computer program code of the computer program product of the first aspect outlined above.

A further aspect of the invention is a computer-implemented method comprising: accessing a clinical database recording historical ECG signal recordings for a patient population, and epidemiological data for the patient population; clustering waveform features extracted from the historical ECG signal recordings according to morphology characteristics to derive a plurality of ECG waveform morphology clusters; using the epidemiological data in the clinical database, correlating each of the plurality of morphology clusters with epidemiological information associated with the patients to which the ECG waveform features in each cluster correspond; identifying by the correlating one or epidemiological features correlated with each cluster; and creating a data structure associating ECG waveform feature morphology with correlated epidemiological features and transferring the data structure to a datastore. Any of the optional features outlined above in relation to the first aspect of the invention may also be applied or incorporated in the present method aspect of the invention.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 is a flowchart of steps of an example method in accordance with one or more embodiments of the invention;
FIG. 2 illustrates a block diagram of an example system in accordance with one or more embodiments of the invention;
FIG. 3 illustrates a process flow for determining ECG beat morphology clusters in accordance with one or more embodiments of the invention;
FIG. 4 depicts morphology cluster visualization plots for two example morphology clusters;
FIG. 5 schematically illustrates relationships between morphology clusters and correlated epidemiological information;
FIG. 6 depicts a heatmap visualization illustrating correlations between ECG beat clusters and patient comorbidities;
FIG. 7 illustrates a block diagram of an ECG analysis system utilizing a callable software module in accordance with one or more embodiments of the invention; and
FIG. 8 depicts an example graphical user interface for use by a user in accessing correlated epidemiological information for user-indicated waveform features of a patient ECG.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for identifying clinically relevant information pertaining to current or predicted future patient status based on inference from patient ECG measurements. The method is based on using a database of ECG signal data and associated epidemiological data to determine correlations between morphological features of ECG signal waveform segments and epidemiological features. ECG waveform features are grouped by morphology into morphology clusters, and each cluster is correlated with epidemiological features. Morphology of new ECG signal data is mapped against the morphology clusters to infer relevant epidemiological features for the patient. The clusters can be updated over time as new ECG data is acquired to reflect changing correlations between ECG feature morphology and epidemiological features.

It is the recognition of the inventors that a system for ongoing correlation of ECG beat morphologies with clinical and epidemiological data has not before been implemented. As a result, informed clinical decisions based on ECG features have not been enabled. The identification of novel digital biomarkers using large-scale ECG data has furthermore not been possible.

Inter alia, embodiments of the present invention may seek to address one of more of the following limitations:
- current ECG interpretation algorithms do not utilize mutual information across patient datasets
- no typical beat morphologies have been defined for sub-populations based on age, sex, or phenotype;
- patients without declared comorbidities are at risk of misdiagnosis during early disease development.

By way of general introductory summary, one or more embodiments of the invention may include any one or more of the following features and functionalities.

In accordance with embodiments of the present invention, beat morphologies from multiple patients may be cross-referenced, and a catalog of ECG morphologies and matched epidemiological data may be generated. Updating of the catalog allows for changing morphologies to be identified as potential indicators of future conditions. Early clinical intervention is therefore facilitated.

At least one set of embodiments of the invention provide a system and method for ECG analysis in which ECG waveform morphologies are correlated with epidemiological attributes. Large-scale clinical databases containing historical ECG and patient data may be used. Previously undetectable associations between waveform features and health conditions may consequently be revealed.

A clustering algorithm may be implemented to group ECG waveform features, and wherein clusters are formed based on morphological similarity. Each cluster may represent a common morphological pattern found across a patient population. Patient data associated with each cluster may be analyzed. One or more epidemiological features linked to each waveform cluster may be identified.

In some embodiments, machine learning techniques may be applied. ECG and patient data may be processed and correlations between waveform morphology and health indicators may be automatically identified. Digital biomarkers and risk factors may be identified wherein these may be indicative of current conditions, future risks, or predispositions. Embodiments of the present invention thus go beyond traditional ECG analysis, which detects only known cardiac abnormalities.

In some embodiments, the system may be adaptive. For example, new ECG recordings and patient information may be continuously integrated. Morphology clusters may be refined as new data are added. In some embodiments, new clusters may be created when novel patterns are detected. This ensures insights remain current and clinically relevant.

A user interface may be included. This may permit a clinician to query the system using waveform features extracted from a patient ECG recording. Morphologically matched morphology clusters may be returned with associated epidemiological correlations. This provides insights into potential risks or underlying conditions of a patient in real time. Clinical decisions may accordingly be supported with timely and relevant information.

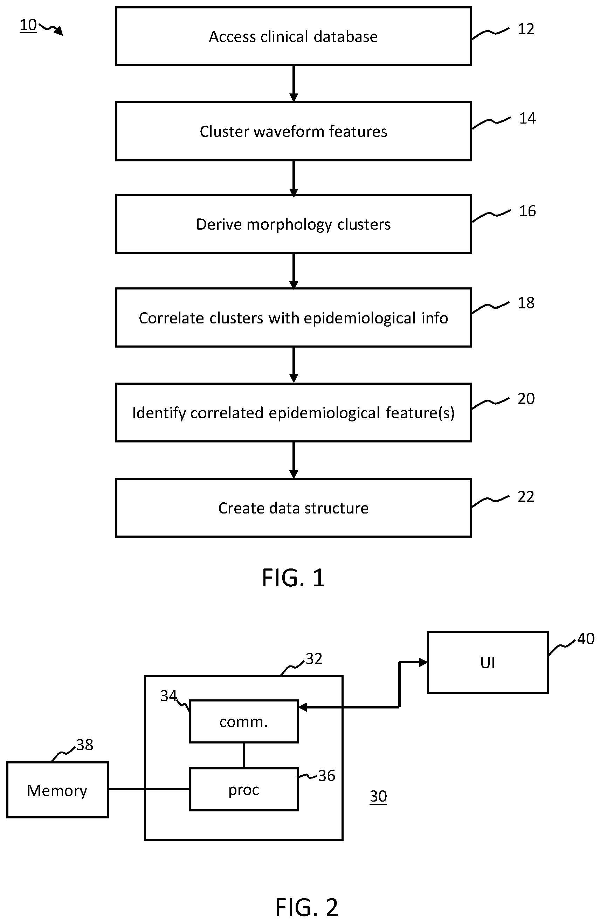

FIG. 1 outlines in block diagram form steps of an example method in accordance with to one or more embodiments. The steps will be recited in summary, before being explained further in the form of example embodiments.

The method 10 comprises accessing 12 a clinical database recording historical ECG signal recordings for a patient population, and epidemiological data for the patient population.

The method 10 further comprises clustering 14 waveform features extracted from the historical ECG signal recordings according to morphology characteristics to derive 16 a plurality of ECG waveform morphology clusters.

The method 10 further comprises, using the epidemiological data in the clinical database, correlating 18 each of the plurality of morphology clusters with epidemiological information associated with the patients to which the ECG waveform features in each cluster correspond.

The method 10 further comprises identifying 20 by the correlating one or epidemiological features correlated with each cluster.

The method 10 further comprises creating 22 a data structure associating ECG waveform feature morphology with correlated epidemiological features and transferring the data structure to a datastore.

In some embodiments, the method may comprise a process of extracting one or more ECG waveform features from each of at least a subset of the historical ECG signal recordings, and performing the clustering 14 of the extracted ECG waveform features. In some embodiments, the process of extracting may comprise segmenting the ECG signals, wherein the ECG waveform features are ECG waveform segments. In some embodiments, the process of extracting may comprise ECG beat segmentation, wherein the ECG waveform features are individual ECG beats, or sections thereof.

As noted above, the method can also be embodied in hardware form, for example in the form of a processing system which is configured to carry out a method in accordance with any example or embodiment described in this document, or in accordance with any claim of this application.

FIG. 2 illustrates a block diagram of a system 30 for ECG analysis. The system 30 includes a processing system 32 that comprises a communication module 34 and one or more processors 36. The system further comprises a memory 38. The system may optionally further comprise a user interface 40. The user interface 40 may comprise a display. The user interface may comprise a user input means, for example a pointer device and/or a touchscreen interface.

The system 30 is configured to execute the method 10 described earlier, with reference to FIG. 1.

The one or more processors 36 may be configured to execute computer program code stored in the memory 38. This computer program code implements the various steps of the method 10.

The communication module 34 enables data exchange between the processing system 32 and the user interface 40.

Optionally, the memory 38 may store the clinical database containing historical ECG signal recordings and epidemiological data, as well as the derived morphology clusters and their associated epidemiological correlations. Alternatively, the clinical database may be stored at a different data storage site.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

As noted above, one step of the method comprises creating a data structure associating ECG waveform feature morphology with correlated epidemiological features.

The data structure may take several forms. In some embodiments, the data structure may be implemented as a relational database table. For example, in one possible implementation of such a table, each row represents a morphology cluster and includes: a cluster identifier, one or more representative waveform morphology features (e.g., average morphology parameters), one or more associated epidemiological features, optionally, one or more correlation scores or probabilities reflecting the statistical strength of each association.

By way of a further example, the data structure may be represented as a graph data model, with nodes representing clusters and epidemiological features respectively, and edges representing statistically derived correlations. Each edge may be weighted based on the correlation value or frequency of co-occurrence in the clinical database.

In further embodiments, the data structure may comprise a matrix format, such as a correlation matrix, with rows corresponding to morphology clusters and columns corresponding to epidemiological features. Each cell in the matrix may store a numerical value representing the strength of the correlation between the corresponding cluster and feature.

Alternatively, the data structure may be stored as a serialized object (e.g., in JSON or XML format), configured for direct use by a query engine within a software module. This enables flexible integration with user interface components and external systems via API communication.

Optionally, in some implementations, the data structure may additionally store metadata, for example including one or more of: time of last update, size and patient count of each cluster, waveform segment definitions (e.g., P wave, QRS complex, T wave), model confidence levels for associations.

As noted above, the method may employ use of a clinical database comprising ECG recordings, each matched to a known patient profile which includes associated epidemiological information such as health conditions and phenotypes.

The ECG signals in the clinical database may originate from a plurality of sources, including both Holter and resting ECG recordings. The ECG recordings may be recordings using different numbers of ECG leads.

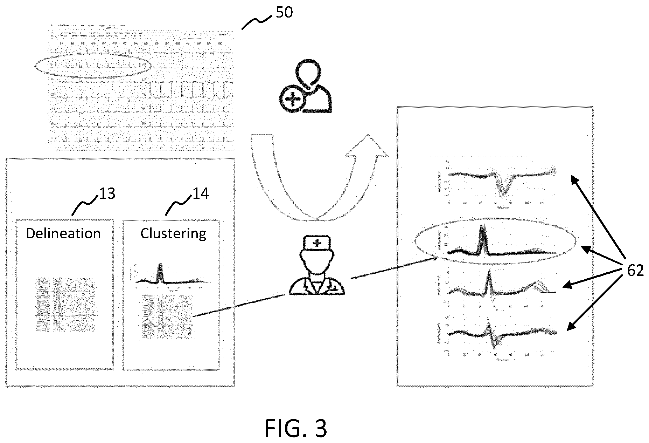

FIG. 3 illustrates an overview of the processing pipeline in accordance with one example implementation. A clinical database 50 stores historical ECG signals 90 associated with patients and the health records of patients. The ECG signals may be processed by a delineation module 13 and a clustering module 14. The delineation module 13 is configured to extract waveform features by segmenting each ECG signal into individual ECG beats. In this example, the ECG beats are the extracted waveform features. The clustering module 14 performs clustering of the extracted waveform features based on morphological similarity, producing morphology clusters 62.

The result is a morphology cluster bank visualized on the right-hand side of FIG. 3. Each morphology cluster 62 in the bank is associated with certain morphology types and corresponding epidemiological information. The bank may optionally be made accessible to the practitioner via a user interface 40.

In some embodiments, the method comprises applying ECG beat segmentation to each ECG signal recording of the clinical database to extract individual ECG beats, wherein previously mentioned ECG waveform features may correspond to these individually segmented beats or subsections thereof.

In some embodiments, the extraction of ECG waveform features may be performed through a multi-step process utilizing a delineation algorithm. A delineation algorithm may be applied to the ECG signal to identify and segment individual heartbeats. The delineation process typically involves the detection of fiducial points, such as the onset, peak, and offset of the P wave, QRS complex, and T wave. Various techniques may be employed for this purpose, including wavelet transform-based methods, adaptive thresholding, or machine learning approaches. The algorithm iterates through the ECG signal, marking these key points and effectively partitioning the continuous recording into discrete beat segments. The segmented beats may correspond to the extracted ECG waveform features mentioned previously.

Subsequently, one or more predetermined morphological features may be extracted from each waveform feature. Clustering may then be performed based on these extracted morphological features. For example, the morphology clusters may be formed by grouping extracted ECG beats with similar morphological characteristics. By way of example, the morphological features used for clustering may include one or more of: P wave characteristics, QRS complex features, and T wave features. Variations in any one or more of: P wave morphology (e.g., sinus vs. ectopic), QRS complex duration (e.g., conduction disorders), amplitude changes (e.g., obesity, pediatric vs. adult profiles), and/or morphology (e.g., ventricular hypertrophy) may optionally be used in defining cluster types.

The clustering may be performed by application of any suitable clustering algorithm, for example k-means clustering or hierarchical clustering, to group similar morphologies.

By way of example, the one or more morphological features extracted from each beat may then be compiled into a feature vector for each beat, which serves as the input for subsequent clustering and analysis steps. This beat-level feature extraction allows for a detailed characterization of ECG morphology, enabling the identification of subtle variations that may be indicative of different physiological or pathological states.

By way of one example implementation, the morphological characteristics may be represented as multidimensional vectors, wherein each dimension corresponds to a specific morphological characteristic such as P wave amplitude, QRS complex duration, or T wave symmetry. Subsequently, the extracted waveform feature morphologies may be clustered using one or more clustering algorithms.

In some embodiments, the clustering may be performed using one or more unsupervised machine learning algorithms. The clustering process may be performed in the high-dimensional feature space, where similar morphologies are grouped together based on their Euclidean or Mahalanobis distances for example. The resulting clusters may be characterized by their centroids, which represent the average morphology of all waveforms within each cluster.

The clustering algorithm may create a morphology cluster bank in which all detected ECG beats from the database are grouped. Each cluster may correspond to a distinct morphology type and may be labelled accordingly. Each cluster may be associated with an average morphology representing an average morphology of the beats included in the cluster.

The method further comprises a step of correlating 18 each of the plurality of morphology clusters with epidemiological information associated with the patients to which the ECG waveform features in each cluster correspond.

The morphology clusters are linked in the backend to correlated patient data. This may include epidemiological features such as age, sex, ethnicity, and/or diagnosed comorbidities or health conditions. These conditions include, but are not limited to, cardiovascular diseases, cardiac syndromes, metabolic disorders (e.g., diabetes, hypercholesterolemia), and chronic diseases.

In some embodiments, upon upload of a new ECG signal to the platform, the signal may be automatically processed. One or more ECG beats extracted from the new ECG signal may be either assigned to an existing morphology cluster or a new cluster may be created if the morphology is sufficiently distinct from the morphology of existing clusters.

In some embodiments, the morphology cluster bank may be dynamic. As additional ECG data is incorporated, existing clusters may be refined, new clusters added, or existing clusters subdivided into more specific subgroups. A feedback loop may this be enabled wherein each newly processed patient contributes to and benefits from the morphology cluster bank. The knowledge base thereby evolves over time. This iterative process supports continuous improvement of the clustering accuracy. This functionality will be described in greater detail to follow.

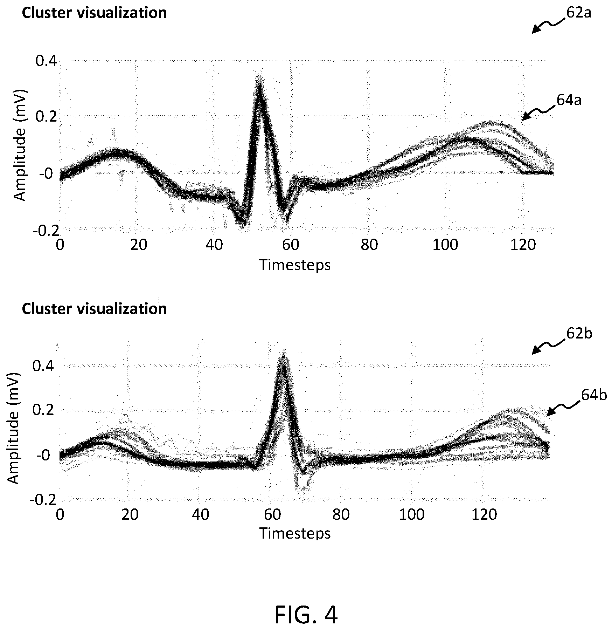

FIG. 4 provides visual examples of two morphology clusters 62a and 62b. The upper plot shows a first cluster 62a, composed of a set of waveform features 64a. This cluster has been found to correlate with conditions such as diabetes and tobacco use. The waveform features 64a within the cluster are plotted in superposition with one another and centered around the QRS complex. This cluster 62a exhibits a widened QRS complex, which may be attributed to QT interval variability resulting from metabolic effects such as fat accumulation. Additionally, ST segment abnormalities in the average beat morphology may correlate with tobacco-induced cardiac changes.

The lower plot shows a second morphology cluster 62b, composed of a second set of waveform features 64b. This cluster is correlated with coronary artery disease. Again, waveform features 64b within this cluster exhibit a similar morphology, and are plotted in superposition with one another and centered around the QRS complex to illustrate the similarity. Variations in T-wave morphology-such as inversion or flattening-are observed across the waveform features of this cluster 62b. Such morphological changes are consistent with known markers of ischemia and support the association with coronary artery pathology.

The morphology is distinguished from that of the first cluster 62a. These examples illustrate how morphology clusters are distinguishable and linked to specific health conditions.

Each waveform feature 64a, 64b contributes to defining the overall or average morphology of its cluster.

With regard to the step of correlating 18 each of the plurality of morphology clusters 62 with epidemiological information 72 associated with the patients to which the ECG waveform features 64 in each cluster correspond, and the step of identifying 20 by the correlating one or more epidemiological features 74 correlated with each cluster, this may be implemented as follows.

**In** one embodiment, the correlation step may comprise extracting patient-specific epidemiological information 72 from the clinical database 50. For each morphology cluster 62, the set of patients whose ECG waveform features 64 are assigned to the cluster is identified. The epidemiological profiles of these patients are aggregated and analyzed to detect statistical associations between the cluster and various epidemiological features 74.

Statistical analysis techniques may be employed to perform this correlation. These techniques may include, but are not limited to, Pearson correlation, chi-square tests, mutual information scoring, odds ratios, or logistic regression. **In** some embodiments, multivariate analysis may be used to account for confounding variables, such as age or sex, when identifying associations with health conditions, such as hypertension, diabetes, heart failure, or genetic predispositions.

**In** another embodiment, machine learning classifiers may be trained to predict the presence or absence of a given epidemiological condition based on waveform morphology characteristics. The predictive power of the model for each condition, when applied across waveform features in a specific cluster, may be used to assign correlation scores to that cluster. Clusters demonstrating high predictive accuracy for a given condition are then tagged with that epidemiological feature 74.

The identification step may involve applying a threshold to the correlation scores. For example, only features whose correlation score with the cluster exceeds a predefined threshold (e.g., 0.6 or higher) are retained as being meaningfully correlated. **In** other embodiments, ranking or weighting of features may be used instead of strict thresholds, thereby allowing presentation of a ranked list of associated conditions.

To enhance robustness, the correlations may be validated using bootstrapping, cross-validation, or repeated sampling of the patient dataset. This ensures that identified correlations reflect repeatable patterns in the underlying data.

**In** some embodiments, unsupervised dimensionality reduction techniques such as principal component analysis (PCA) or t-distributed stochastic neighbor embedding (t-SNE) may be used to visualize the relationship between clusters and patient-level epidemiological data. These techniques may support discovery of latent patterns and improve interpretability.

The output of this correlation process is a mapping between each morphology cluster 62 and a subset of epidemiological features 74 most representative of the patient group associated with that cluster. This mapping is stored in the data structure created in step 22 of the method 10 and may be used subsequently to provide real-time inference, decision support, or cluster refinement.

The resulting association between morphology and epidemiological features may further be used to label each cluster with one or more descriptive terms (e.g., "diabetes-associated morphology", "coronary risk indicator", or "pulmonary-disease beat type"), enhancing interpretability.

The identified epidemiological features 74 may for example include predictors of at least one extant health condition, predictors of future health conditions, and/or indicators of predisposition to one or more health conditions. Further, the patient epidemiological information 72 may comprise one or more of age, sex, medical history, lifestyle factors, genetic data, or diagnosed health conditions.

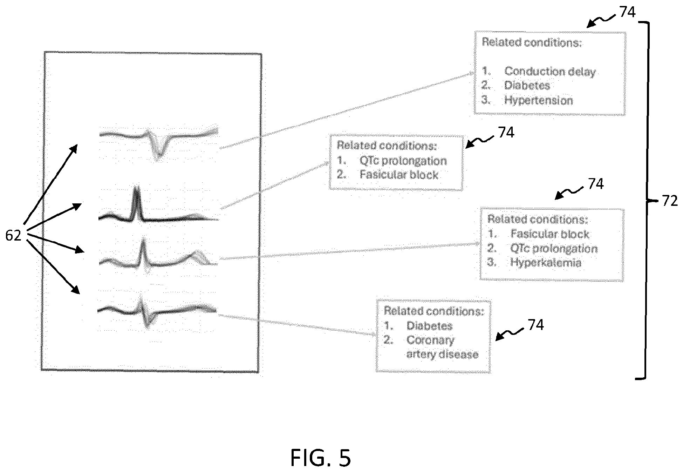

FIG. 5 schematically illustrates an example mapping between morphology clusters 62 and correlated epidemiological information 72. Each morphology cluster 62 is associated with a corresponding set of correlated epidemiological features 74. These features 74 may for example represent specific health conditions statistically linked to the waveform features within the given cluster.

The morphology clusters 62 are derived from previously segmented and clustered ECG waveform features 64, processed by the method 10 using ECG signals 90 stored in the clinical database 50. The mapping shown in FIG. 5 serves as an interpretive tool, allowing practitioners to assess correlations between beat morphologies and underlying patient health conditions.

**In** some embodiments, the correlation 18 process may be repeated periodically or triggered dynamically upon appending of new ECG recordings into the clinical database 50. **In** such cases, both the clusters and their associated epidemiological features 74 may be updated automatically, ensuring that the system continuously adapts to the most recent data and reflects evolving clinical knowledge.

Optionally, the user interface 40 may permit a clinician user to explore the different morphology clusters 62, for example one at a time. This facilitates contextual reflection on clinical conditions potentially represented within the electrocardiogram morphology. A cluster average beat morphology may exhibit patterns, such as QRS widening or T-wave variations, which can be indicative of underlying pathophysiological conditions.

**In** some embodiments, the user interface 40 may be configured to permit a clinician user to view example ECG beats of other patients in the database 50 whose beats have been assigned to the same cluster 62 as a current patient. This comparative view enables a practitioner to identify patterns shared with the currently investigated patient's ECG and to detect deviations. A mismatch between the patient's beat morphology and that of the assigned cluster may reveal latent or emerging cardiovascular markers.

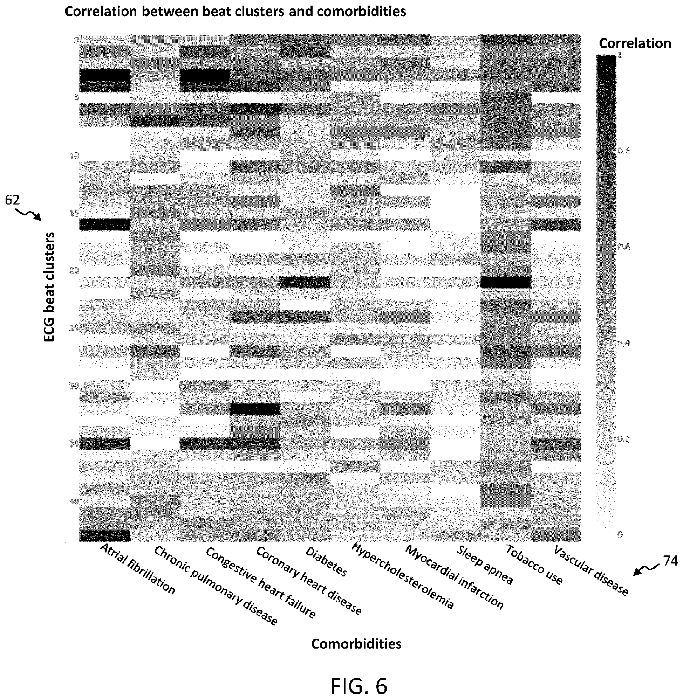

With reference to FIG. 6, a correlation matrix 70 (otherwise known as a correlation 'heat map') is illustrated. The correlation matrix 70 visually represents the relationships between individual morphology clusters 62 and various correlated epidemiological features 74 derived from patient data within the clinical database 50.

Each row in the matrix corresponds to an individual morphology cluster 62. Each column corresponds to a comorbidity or other epidemiological feature 74. The color intensity of each cell indicates the degree of correlation between the corresponding cluster and condition. Darker shades represent stronger correlations, while lighter shades indicate weak or absent correlations.

The correlation matrix 70 may support the step of identifying 20 correlated epidemiological information 72 for each morphology cluster 62.

In some embodiments, the method 10 may comprise updating the morphology clusters 62 dynamically as new data is incorporated. In some embodiments, the method comprises, responsive to an update of the clinical database 50, identifying one or more newly added ECG signal recordings. From each newly added ECG signal, one or more waveform features 64 are extracted. Each extracted feature is assigned to one or more existing morphology clusters 62 based on morphology similarity. The correlated epidemiological information 72 for each cluster may then be updated. This updating may include repeating the step of correlating 18 each morphology cluster 62 with the epidemiological features 74 of the patients whose ECG waveform features 64 have been assigned to that cluster.

**In** some embodiments, the method 10 may comprise performing an evaluation of morphology similarity between each extracted waveform feature 64 of a newly added ECG signal and an average morphology associated with each existing morphology cluster 62. If no match exceeds a predefined similarity threshold, a new morphology cluster 62 may be created to accommodate the unmatched feature. The new cluster is assigned an average morphology, and the similarity measure between the new waveform feature and the new cluster is validated to exceed the pre-defined threshold.

By way of example, the implementation of such a morphology similarity evaluation between newly extracted waveform features and existing morphology clusters may be performed as follows. Initially, a representative average morphology may be computed for each existing morphology cluster. This average morphology may be derived by aligning the waveform features within each cluster and calculating the mean amplitude at each time point across all aligned waveforms. The resulting average morphology may serve as a template against which new waveform features can be compared.

For each newly extracted waveform feature, a similarity score may be calculated with respect to the average morphology of each existing cluster. This similarity assessment may be performed using various metrics, such as the Pearson correlation coefficient, dynamic time warping distance, or wavelet-based similarity measures. The chosen similarity metric may be applied between the new waveform feature and each cluster average morphology, resulting in a set of similarity scores.

A predefined similarity threshold may be established to determine whether a new waveform feature sufficiently matches any existing cluster. This threshold may be set based on empirical analysis of the dataset or through statistical methods such as receiver operating characteristic (ROC) curve analysis. If the highest similarity score for a new waveform feature does not exceed this threshold when compared to all existing clusters, a new morphology cluster may be created.

The process of creating a new morphology cluster may involve initializing the cluster with the unmatched waveform feature as its sole member. The average morphology for this new cluster may be set to the morphology of the unmatched feature itself. As additional waveform features are processed, they may be compared against this newly created cluster along with the pre-existing clusters, potentially leading to the growth of the new cluster over time.

To validate the creation of the new cluster, the similarity measure between the initiating waveform feature and the new cluster's average morphology (which is identical to the feature itself at this stage) may be computed. This similarity measure should, by definition, exceed the pre-defined threshold, confirming the appropriateness of creating the new cluster. This validation step may serve as a safeguard against potential errors in the cluster creation process.

Further, in some embodiments, a cluster refinement operation may be performed. This may comprise merging two or more morphology clusters 62 or splitting a single cluster into multiple refined clusters. This refinement process may be triggered periodically or in response to specific events, such as the addition of a significant number of new waveform features or the detection of changes in the overall distribution of morphologies. This operation may be executed in response to a morphology similarity assessment conducted across the waveform features 64 of the affected clusters.

The implementation of a cluster refinement operation may be performed as follows.

To assess the potential for merging clusters, a pairwise similarity analysis may be conducted between the average morphologies of all existing clusters. This analysis may utilize the same similarity metrics employed in the initial clustering process. Clusters whose average morphologies exhibit similarity scores above a predefined merging threshold may be identified as candidates for merging.

The merging process may involve combining the waveform features from the identified clusters into a single, larger cluster. A new average morphology may be computed for the merged cluster by aligning and averaging all constituent waveform features. The metadata associated with the original clusters, such as correlations with epidemiological features, may be aggregated and updated to reflect the characteristics of the merged cluster.

Conversely, the splitting of clusters may be initiated when significant heterogeneity is detected within a single cluster. This heterogeneity may be quantified by calculating the within-cluster variance or by applying dimensionality reduction techniques such as principal component analysis (PCA) to visualize the distribution of waveform features within the cluster.

If the heterogeneity within a cluster exceeds a predefined threshold, a splitting procedure may be executed. This procedure may involve applying a clustering algorithm, such as k-means or hierarchical clustering, specifically to the waveform features within the heterogeneous cluster.

Following the splitting process, new average morphologies may be computed for each resulting sub-cluster. The epidemiological correlations associated with the original cluster may be reevaluated for each sub-cluster, potentially revealing more specific relationships between particular morphology subtypes and patient characteristics.

Both the merging and splitting operations may be followed by an update of the data structure that associates ECG waveform feature morphologies with correlated epidemiological features. This update may ensure that the refined cluster structure accurately reflects the current understanding of the relationships between ECG morphologies and patient health characteristics.

FIG. 6 illustrates the refinement of clusters with reference to the correlation matrix 70. Certain clusters exhibit high correlation with multiple comorbidities, thereby limiting specificity. For example, cluster 35 shows high correlation with atrial fibrillation, congestive heart failure, and coronary artery disease. **In** such cases, a splitting operation may be triggered to separate overlapping features into distinct morphology clusters 62.

**In** contrast, clusters 7 and 27 exhibit similar correlation profiles across multiple comorbidities. Both clusters show strong correlation with chronic pulmonary disease and low correlation with unrelated conditions. These clusters may therefore be merged, as they reflect morphological redundancy and are considered mimics of one another.

The system may iteratively refine the clusters to optimize the organization of the morphology cluster bank. An aim may for example be to minimize overlap between unrelated conditions and maximize the predictive value of each cluster. Through repeated refinement, the morphology clusters 62 evolve to represent discrete, clinically meaningful units of morphology that may serve as digital biomarkers.

Once deployed, the system may be configured to repeatedly or continuously adapt the morphology clusters 62 based on incoming ECG recordings from both existing and new patients. The method may utilize up to millions of ECG signals to construct robust families of beat morphologies. This large-scale aggregation improves resistance to outliers and enhances the reliability of morphological classification.

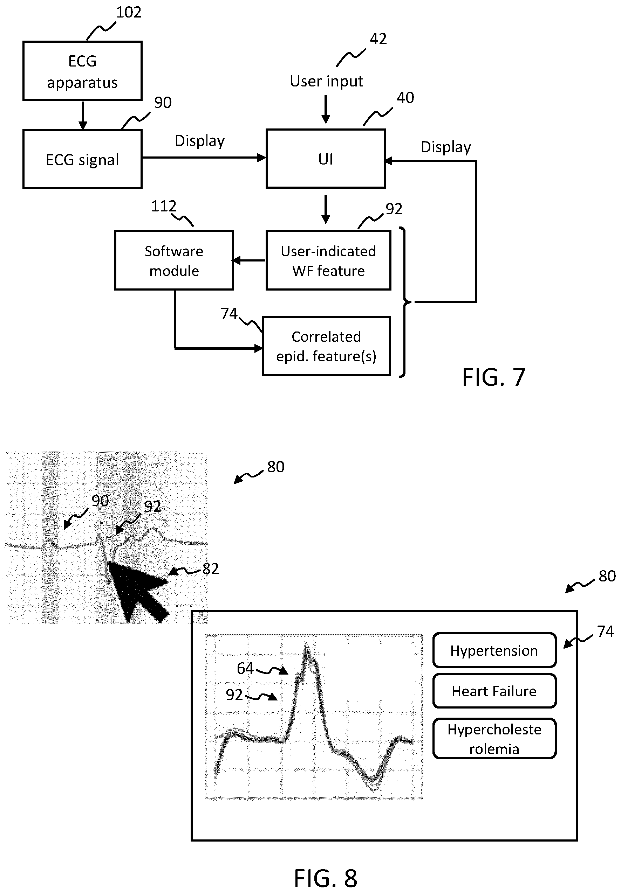

With reference to FIG. 7 and FIG. 8, there is shown an implementation of a method 10 for real-time inference of epidemiological features 74 from a measured ECG signal 90 for a patient. The method enables inference of one or more epidemiological features 74 associated with a patient based on the morphology of ECG waveform features 64 of the measured ECG signal.

An ECG apparatus 102 may output a measured ECG signal 90, responsive to application of measurement electrodes of the apparatus to a patient.

A visual representation of the ECG signal 90 may be displayed to a user (e.g. the user is assumed to be a clinician in this case) via a display device comprised by a user interface 40. This allows the user to review the ECG information and perform interpretation and potentially make clinical decisions based on the information.

The data structure generated through embodiments of the present invention allows for a user to gain additional insights corresponding to the patient based on the ECG signal 90.

The user interface 40 may permit the user to electronically interact with the data structure to determine one or more epidemiological features associated with the ECG signal obtained for the patient. For example, a processing system to which the user interface 40 is coupled may be configured to: receive the measured ECG signal 90 of the patient, extract one or more waveform features 64 from the signal, and match each of the extracted waveform features to one or more morphology clusters 62 in the data structure. Based on this morphological matching, one or more epidemiological features 74 correlated with the ECG signal may be identified. The matching step may for example be performed by comparing the morphology of the extracted waveform feature 64 with that of the morphology clusters 62. This comparison may be performed in real time, enabling instantaneous inference of likely health conditions from observed beat morphology.

One way to implement this functionality is to generate, using the data structure, a software module having a query processing function which uses the generated data structure. The software module may be operable to receive a query indicative of a waveform feature morphology and be operable to output a response indicative of one or more correlated epidemiological features, using the generated data structure. This allows for interfacing between the data structure and a user interface.

For example, in some embodiments, one or more waveform features may be extracted from the received ECG signal 90, and the user interface 40 may be configured to send a query to the software module indicative of a morphology of the waveform feature, and wherein the software module generates and sends back to the user interface a response indicative of one or more correlated epidemiological features, using the generated data structure.

In some embodiments, the user interface 40 may be configured to permit the user to provide a user input 42 indicating one or more individual waveform features of the received ECG signal 90. The software module may then be queries based on the user-indicated waveform features.

For example, with reference to FIG. 8, a user input 42 may be received via the user interface 40, enabling identification of a user-indicated waveform feature 92. The user interface 40 is configured to query the software module 112 based on the user-indicated waveform feature 92 and the software module is configured to identify a morphology cluster to which a morphology of the user-indicated waveform feature is closest matched and to output based on this an indication of the one or more epidemiological features 74 correlated with the morphology cluster based on the data structure. This information may be transmitted back to the user interface 40 as a response message from the software module 112, and the user interface may be configured to display an indication of the identified one or more epidemiological features correlated with the waveform feature based on the response. These may be displayed alongside a visual representation of the ECG signal 90 and/or the user-indicated waveform feature(s) 92 of the ECG signal.

By way of example, the user input 42 may take the form of a user pointer click or cursor hover. For example, in some embodiments, the user interface 40 may be configured to detect the user-indicated waveform feature based on detecting a user cursor hover above the relevant waveform feature of the displayed ECG signal.

FIG. 8 illustrates an example graphical user interface. The user interface comprises a display 80 which presents a visual representation of the ECG signal 90 which incorporated one or more individual waveform features 92 (e.g. ECG beats of the ECG signal). The user may use a pointer device of the user interface (e.g. a mouse or a touchscreen) to move a cursor position relative to the displayed ECG signal. The user may move the cursor to hover over a waveform feature 92 of interest, and this may trigger the previously described process of querying the software module 112 in accordance with the waveform feature which the cursor is hovered over. When the user-indicated waveform feature 92 is detected, a query is sent to the software module 112. The response from the software module includes a set of correlated epidemiological features 74 which are then displayed to the user.

To provide further context to a user, in some embodiments, once the closest matching morphology cluster is identified for the user-indicated waveform feature 92, the system may be configured to display an average morphology waveform representative of an average morphology of the cluster as an overlay superimposed atop the user-indicated waveform feature. This helps the user identify any morphological deviations. This may provide further clinical insight.

**In** other words, the method may comprise matching the user-indicated waveform feature 92 to one of the morphology clusters, determining an average morphology waveform for the matching morphology cluster, and, using the user interface 40, displaying the average morphology waveform as an overlay superimposed atop the user-indicated waveform feature.

This allows for visual comparison between the analyzed waveform feature (e.g. ECG beat) and its corresponding cluster average.

This functionality is illustrated in FIG. 8. As shown in FIG. 8 (bottom), when a waveform feature 92 is selected, the user interface 80 may be configured to display an overlaid waveform 64 that represents the average morphology for the matching cluster 62. Alongside the waveform, a list of the most strongly correlated epidemiological features 74 - such as hypertension, heart failure, or hypercholesterolemia - is shown.

**In** some embodiments, the method optionally comprises displaying an indication of the likelihood associated with each identified epidemiological feature 74, thereby quantifying the strength of the correlation between the user-indicated waveform feature 92 and potential patient health conditions.

Embodiments of the invention described above employ a processing system. The processing system may in general comprise a single processor or a plurality of processors. It may be located in a single containing device, structure or unit, or it may be distributed between a plurality of different devices, structures or units. Reference therefore to the processing system being adapted or configured to perform a particular step or task may correspond to that step or task being performed by any one or more of a plurality of processing components, either alone or in combination. The skilled person will understand how such a distributed processing system can be implemented. The processing system includes a communication module or input/output for receiving data and outputting data to further components.

The one or more processors of the processing system can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

**In** various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. **In** the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer program product comprising computer program code configured, when run on a processor, to cause the processor to perform a method, wherein the method (10) comprises:
accessing (12) a clinical database (50) recording historical ECG signal recordings for a patient population, and epidemiological data for the patient population;
clustering (14) waveform features extracted from the historical ECG signal recordings according to morphology characteristics to derive (16) a plurality of ECG waveform morphology clusters (62);
using the epidemiological data in the clinical database, correlating (18) each of the plurality of morphology clusters with epidemiological information associated with the patients to which the ECG waveform features in each cluster correspond;
identifying (20) by the correlating one or epidemiological features correlated with each cluster; and
creating (22) a data structure associating ECG waveform feature morphology with correlated epidemiological features and transferring the data structure to a datastore.

2. The computer program product of claim 1, wherein the one or more epidemiological features include one or more of:
a predictor of at least one extant health condition;
a predictor of at least one future health condition; or
a predictor of at least one predisposition to a health condition.

3. The computer program product of claim 1 or 2, wherein the method further comprises:
receiving a measured ECG signal of a patient;
matching each of one or more waveform features of the received ECG signal to one or more of the morphology clusters;
identifying, based on the matching, one or more epidemiological features correlated with the ECG signal of the patient.

4. The computer program product of any preceding claim, wherein the method comprises applying ECG beat segmentation to each of the ECG signal recordings of the clinical database to extract individual ECG beats, and wherein the ECG waveform features correspond to individually segmented ECG beats, or sections thereof.

5. The computer program product of any preceding claim, wherein the clustering comprises extracting from each waveform feature one or more predetermined morphological features and clustering the waveform features based on the extracted morphological features.

6. The computer program product of claim 5, wherein the one or more pre-determined morphological features comprise at least one of: P wave characteristics, QRS complex characteristics, and T wave characteristics.

7. The computer program product of any preceding claim, further comprising:
responsive to an update of the clinical database, identifying one or more ECG signal recordings newly added to the database;
extracting one or more waveform features from the one or more newly added ECG signal recordings and assigning the extracted waveform features to one or more of the morphology clusters based on morphology;
updating the correlated epidemiological information for the one or more morphology clusters.

8. The computer program product of any preceding claim, further comprising:
responsive to an update of the clinical database, identifying one or more ECG signal recordings newly added to the database;
extracting one or more waveform features from the one or more newly added ECG signal recordings;
evaluating a morphology similarity measure between morphology of each of the extracted waveform features and an average morphology associated with each of the morphology clusters;
responsive to the similarity measure for a given extracted waveform feature being below a predetermined threshold for all of the morphology clusters, adjusting the morphology clusters to include a new morphology cluster which includes at least the given waveform feature.

9. The computer program product of any preceding claim, further comprising performing a cluster refinement operation comprising merging two or more morphology clusters or splitting one or more morphology clusters,
optionally wherein the cluster refinement operation is performed responsive to a morphology similarity assessment applied to waveform features of the plurality of morphology clusters.

10. The computer program product of any preceding claim, wherein the patient epidemiological data includes one or more of the following epidemiological features of each patient: age, sex, medical history, lifestyle factors, genetic information, and diagnosed health conditions.

11. The computer program product of any preceding claim, wherein the method further comprises generating a software module having a query processing function which uses the generated data structure, wherein the software module is operable to receive a query indicative of a waveform feature morphology and is operable to output a response indicative of one or more correlated epidemiological features, using the generated data structure.

12. The computer program product of claim 11, wherein the method comprises:
receiving an ECG signal;
displaying the received ECG signal using a user interface;
receiving from the user interface indication of a user-indicated waveform feature comprised by the ECG signal;
querying the software module based on the user-indicated waveform feature;
receiving a response from the software module; and
displaying using the user interface an indication of one or more epidemiological features correlated with the waveform feature based on the response.

13. The computer program product of claim 12, wherein the user interface is configured to detect the user-indicated waveform feature based on detecting a user cursor hover above the waveform feature of the displayed ECG signal.

14. The computer program product of claim 12 or 13, wherein the method further comprises:
matching the user-indicated waveform feature to one of the morphology clusters;
determining an average morphology waveform for the matching morphology cluster; and
using the user interface, displaying the average morphology waveform as an overlay superimposed atop the user-indicated waveform feature.

15. A processing system (32) comprising one or more processors (36) configured to execute the computer program code of the computer program product of any preceding claim.
